# EUROPEAN PATENT APPLICATION

(11) **EP 4 840 413 A2**
(43) Date of publication of application: **18.11.2026**
(21) Application number: 26178230.4
(22) Date of filing: 12.05.2026
(51) Int. Cl.: A61B 17/86, A61B 17/68, A61B 17/72, A61B 17/80

(54) **ANKLE FIXATION SYSTEM**

(30) Priority: 16.05.2025 US 202563806845 P
(71) Applicant: Stryker European Operations Limited, Carrigtwohill T45H X08 (IE)
(72) Inventor: ZANDER, Nils, 24340 Eckernförde (DE); SHORABH, Sunny, 201001 Uttar Pradesh (IN)
(74) Representative: Maiwald GmbH

(57) **Abstract**

A system for treating a syndesmosis injury comprising a syndesmosis pin having a head and a shaft and an intramedullary nail configured for implantation in a fibula having a slot sized to receive the shaft for an improved system for treating fibular fractures involving a syndesmotic injury.

## Description

### CROSS-REFERENCE TO RELATED APPLICATIONS

The present application claims the benefit of the filing date of U.S. Provisional Application No. 63/806,845, filed May 16, 2025, the disclosure of which is hereby incorporated herein by reference.

### BACKGROUND OF THE INVENTION

Open reduction and internal fixation ("ORIF") surgery is used to repair broken bones when more conservative methods such as splints and casts are not suitable due to the severity of the break and/or extreme misalignment of the bones. During an ORIF procedure, surgeons bring pieces of broken bones back into proper alignment (*i.e.,* reduction) and then use hardware such as nails, screws and washers to secure the fragments together (*i.e.,* fixation) and facilitate proper healing.

A typical ORIF procedure may include reducing the bone fragments and inserting an intramedullary ("IM") nail into the medullary cavity of a bone as an internal splint to provide durable yet flexible structural support, thereby holding the fracture in proper alignment as the bone heals. The nail shares load with the bone, and reduces the required inactivity time needed before patients can resume full use of their extremities. Screws are typically placed through holes formed in the nail and may also pass through a bone plate placed on the outside of the bone.

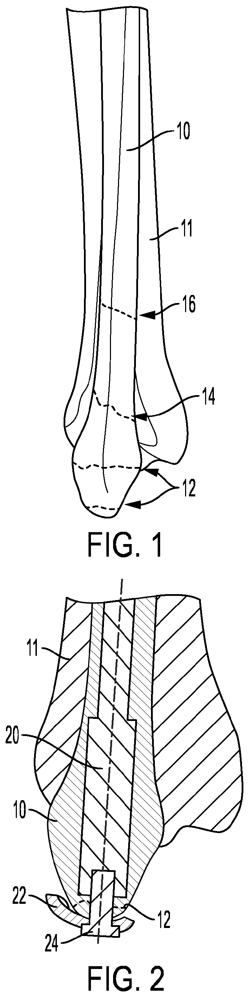

The use of IM nails in ORIF procedures to repair fibula fractures involve specific factors not seen in other bones. FIG. 1 illustrates a distal fibula 10 and tibia 11. Common fibular fractures are also shown - a distal/avulsion fracture (Weber A) 12, a neck fracture (Weber B) 14 and a shaft fracture (Weber C) 16. IM nails are typically utilized for Weber B and C fractures. However, a combination of a Weber B or C fracture and a Weber A fracture are very challenging to treat with an IM nail. Controlling reduction of fractures using an IM nail is also challenging for even Weber B and C type fractures.

Another potential complication involves syndesmotic injuries, which are injuries that cause damage to the ligament that connects the distal fibula and tibial notch. While syndesmotic injuries can be isolated injuries as a result of high ankle sprain, they often accompany shaft fractures or neck fractures of the fibula and tibia following trauma to the ankle. Typical treatments of syndesmotic injuries involve fixing a horizontal screw to connect the fibula, tibia, and, if present or required by other accompanying fractures, an IM nail after reduction of the joint. However, this method typically relies on the grip between the threads of the screw and the bones for fixation, creating tremendous stress and an overly rigid fixation. Because of the high mobility of the ankle, the syndesmosis joint is often subject to load bearing and sheering movement even during relative inactivity. As a result, this method has the potential to create damage in the bone. It is also possible that the screws can shear under the load, not only losing fixation on the joint, but also creating pieces of hardware that are difficult to remove from the patient's bones.

An alternative to the horizontal screw method involves the use of suture buttons that are secured on the medial side of the tibia and lateral side of the fibula to cinch the two bones together. However, this method often increases the costs of the surgery significantly, and adds more technical difficulties both due to the additional instrumentation needed and the difficulty of the technique. Suture button systems may also cause more irritation on surrounding soft tissue, and result in a less stable fixation as compared to the aforementioned screw method. Moreover, because it is still a high-tension fixation, the suture has the potential to loosen or break under the loads at the syndesmotic joint.

Thus, there exists a need for an improved system and method for treating fibular fractures involving a syndesmotic injury.

### SUMMARY OF THE INVENTION

An aspect of the invention is to address the aforementioned difficulties of ankle trauma injury treatment, and to provide a solution that reduces post-surgical complication and promotes optimal healing.

The proposed invention incorporates customizable distal washer plates that are anatomically shaped to be patient and injury specific in addition to traditional intramedullary nail fixation methods. The addition of these washer plates provides for improved distal fracture management, especially for distal and avulsion fractures where the remaining distal bone fragment may be small in size or frail, while preserving the benefits of intramedullary nail fixation methods. The large surface area of the washer plates and the conformity of the plates to the bones serve to minimize localized stress concentrations, preserving the structural integrity of the bone fragments.

Depending on the size and condition of the distal bone fragment of the fractured fibula, the anatomically shaped washer may be standard sized, extended, or extra extended, with hooks or without hooks. The longer and better condition the distal piece has, the longer washer may be to maximize conformation of the system with the patient's fibula, to thereby reduce stress created by nail locking caps, to accommodate for more nail locking caps, and to provide more insertion points for nail locking caps. Surgeons have freedom to create more patient-specific and fracture-specific treatment, improving stability and stress distribution of the fixation system, which ultimately reduces complications both during surgery and post-surgery, and promotes optimal healing.

Additionally, the present invention provides for a syndesmosis pin to be used in combination with a patient or injury specific slot in the intramedullary nail to accommodate for ankle trauma cases where syndesmosis injuries are present. The slot is sized such that the syndesmosis pin can move freely within the slot after insertion, and the angle of the slot is created to accommodate the insertion angle needed for the specific patient and injury. The corresponding intramedullary nail can be selected from a series of pre-slotted intramedullary nails or created by the user on an intramedullary nail without pre-slots. The slots can be horizontal, vertical, or slanted. The slots create flexibility in pin positioning which is crucial to preserve proper alignment and reduction of the bones.

In use, after an intramedullary nail is inserted into the patient's fibula, a channel for insertion of a syndesmosis pin is created through the patient's fibula, the inserted intramedullary nail and the patient's tibia. The channel is sized such that a shaft of the syndesmosis pin is able to flex under loads within the channel after insertion. The syndesmosis pin is inserted from the patient's fibula through the patient's tibia. The head of the syndesmosis pin is larger than the channel so that the pin is secured by the head on the lateral side of the fibula. The larger diameter of the slots relative to the syndesmosis pins allow the syndesmosis pins more degrees of flexion during ankle motion as compared to rigidly connected threaded screws, and can absorb more stress without breaking. Due to the syndesmosis pin's fixed length, it is not subject to loosening as suture button systems would be.

The shaft of the syndesmosis pin is smooth with no thread to avoid creating stress risers within the channel. The pin can be made of nitinol or other flexible metals and alloys to achieve inherent flexibility, further enabling the syndesmosis pins to absorb stress and adapt to different loading conditions during ankle movement. The tip of the syndesmosis pin may extend beyond the tibia, and a locking or condyle type washer may be used on the medial side of the tibia to couple with the tibia end of the syndesmosis pin shaft. Gradual compression can be applied through coupling the washer and the syndesmosis pin tip, for example, through threading the washer onto the syndesmosis pin tip or through press fit, securing the fixation. The syndesmosis pin tip may be threaded or tapered to allow for more washer compatibility and precise control over compression.

One aspect of the present disclosure is a system for treating a syndesmosis injury including a syndesmosis pin having a head and a shaft and an intramedullary nail configured for implantation in a fibula having a slot sized to receive the shaft. The slot is sized larger than the shaft of the syndesmosis pin to allow for movement of the syndesmosis pin within the slot.

In certain embodiments, the syndesmosis pin may be flexible, including being constructed of nitinol. The slot may be parallel, perpendicular or neither to a longitudinal axis of the intramedullary nail. The shaft of the syndesmosis pin may be unthreaded or threaded. A lock washer may be configured to be coupled with the syndesmosis pin. The lock washer may be configured to conform to a surface of a tibia.

In accordance with another aspect of the present disclosure, a method of treating a syndesmosis injury may include implanting an intramedullary nail in a fibula, the intramedullary nail having a slot formed therethrough, creating a channel extending through the fibula and a tibia, inserting a syndesmosis pin into the channel and through the slot such that a portion of the syndesmosis pin extends out of the tibia and coupling a lock washer to the portion to create a compression between the fibula and tibia.

In other embodiments of this aspect, the inserting step may include inserting a flexible pin. The slot may be sized larger than the shaft of the syndesmosis pin to allow for free flexing of the syndesmosis pin within the slot. The step of selecting the intramedullary nail from a plurality of intramedullary nails may have differently oriented slots. The differently oriented slots may include slots having longitudinal axes parallel, perpendicular, and neither perpendicular nor parallel to a longitudinal axis of the intramedullary nail. The coupling step may include threading the washer on the portion. The coupling step may include press fitting the washer on the portion. The coupling step may include applying a gradual compression between the fibula and tibia.

Another aspect of the present disclosure is a system for treating a fracture of a fibula include an intramedullary nail configured for implantation in a fibula, a washer configured to cooperate with a distal end of the fibula, the washer including a first hole, and a nail cap extending through the first hole and connected to the intramedullary nail.

In other embodiments of this aspect, the washer conforms to the distal end of the fibula. The washer may include a second hole configured to receive a bone screw. The system may further include the bone screw. The washer may include a hook for engaging the fibula. The nail cap may be threaded to a distal end of the intramedullary nail.

### BRIEF DESCRIPTION OF THE DRAWINGS

A more complete appreciation of the subject matter of the present invention and of the various advantages thereof can be realized by reference to the following detailed description in which reference is made to the accompanying drawings in which:
FIG. 1 is a perspective view of a distal fibula and tibia illustrating common fibular fractures.
FIG. 2 is a cross-sectional view depicting an IM nail, washer and nail cap in accordance with the present disclosure in conjunction with a distal fibula and tibia.
FIG. 3 is a perspective view of another washer and nail cap in accordance with the present disclosure in conjunction with a distal fibula and tibia.
FIG. 4 is a cross-sectional view depicting the washer and nail cap of FIG. 3 in conjunction with an IM nail and a distal fibula and tibia.
FIGs. 5A-C are different views of the washer, nail cap, screw and IM nail of FIG. 3.
FIG. 6 is a perspective view of another washer and nail cap in accordance with the present disclosure in conjunction with a distal fibula and tibia.
FIG. 7 is a cross-sectional view depicting the washer and nail cap of FIG. 6 in conjunction with an IM nail and a distal fibula and tibia.
FIGs. 8A-C are different views of the washer, nail cap, screw and IM nail of FIG. 6.
FIG. 9 is a cross-sectional view depicting an IM nail, lock washer and flexible pin in accordance with the present disclosure in conjunction with a distal fibula and tibia.
FIGs. 10A-C are views focusing on different syndesmosis pin slots of the IM nail of FIG. 8.

### DETAILED DESCRIPTION

In describing the preferred embodiments of the inventions, specific terminology will be used for the sake of clarity. However, the inventions are not intended to be limited to any specific terms used herein, and it is to be understood that each specific term includes all technical equivalents, which operate in a similar manner to accomplish a similar purpose. In the drawings and in the description which follows, the term "proximal" refers to the end of instrument, or a portion thereof, which is closest to the operator in use, while the term "distal" refers to the end of the instrument, or portion thereof, which is farthest from the operator in use. When referring to the human body, the term "proximal" means closer to the heart, the term "distal" means more distant form the heart, the term "anterior" means towards the front part of the body or the face and the term "posterior" means towards the back of the body. The term "medial" means toward the midline of the body and the term "lateral" means away from the midline of the body.

Referring to FIG. 2, a fibula fixation system includes an IM nail 20, a washer 22 and a nail cap 24. The system is particularly useful for treating avulsion/Weber A fractures in a fibula 10, but does have applicability in conjunction with other bones and in treating other types of fractures. IM nail 20 is shown implanted within fibula 10 and exhibiting a shape suitable for such a use. In this regard, IM nail 20 can take on many different forms, including IM nails similar to the various T2 Alpha and Gamma nails offered by Stryker Corp. or its subsidiaries, and other cylindrical, hollow, solid, stepped, straight, angled or bowed rods. In addition to being suitable for placement within fibula 10, IM nail 20 must include a structure that can cooperate with nail cap 24. The particular IM nail design shown includes a threaded opening at its distal end that can threadably couple with nail cap 24. Other connection mechanisms are contemplated, such as ratchet, friction-fit and the like for both IM nail 20 and cap 24.

Washer 22 is in form of hook plate that has a shape suitable for cooperation with the distal end of fibula 10. Washer 22 includes a hole that nail cap 24 can be placed through. In particular, this hole is sized such that a shaft of nail cap 24 can extend through the plate, but a head of the cap abuts an outer surface. Washer 22 includes one or more hooks that engage a distal portion of fibula 10 to aid in holding the washer in place and creating a stronger, more stable construct. In other embodiments, washer 22 need not include such hooks or may include different structures for engaging the bone. Washer 22, as shown, is contoured to mate with the distal end of fibula 10. It is contemplated that the washer could be bent to better mate with a given bone, or even be patient-specifically designed for an even better mating.

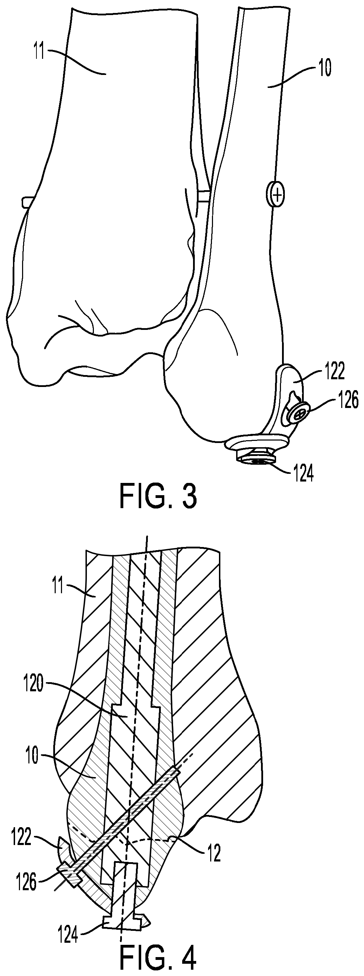

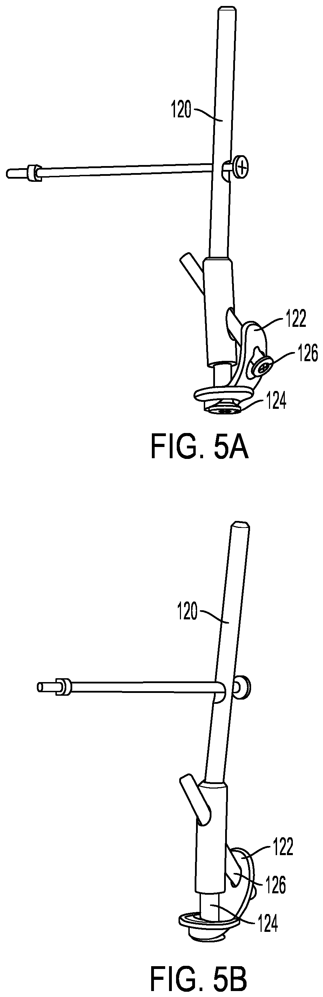

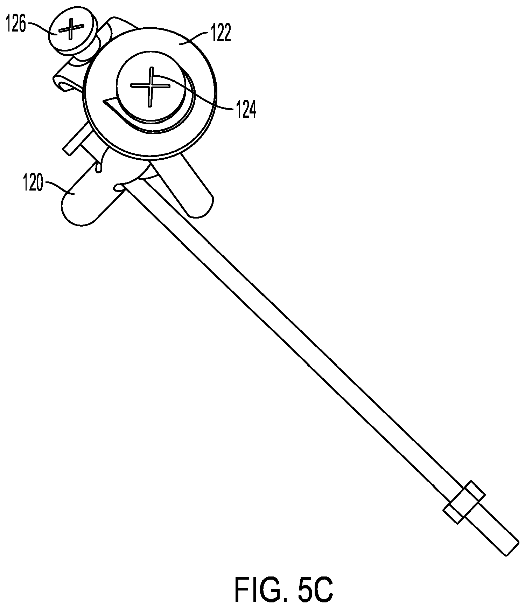

FIGs. 3-5C depict a similar system particularly useful in treating distal/Weber A fractures. Because of the similarities between the components of this system with the above-discussed system, like components are labeled with like reference numerals, but within the 100-series of numbers. For instance, the system of FIGs. 3-5C includes an IM nail 120, a washer 122 and a nail cap 124. The difference between the two systems lies in the washer 122, which is an extended version of the washer described above. Moreover, washer 122 includes a second hole that can receive a compression screw 126 that aids in fracture compression.

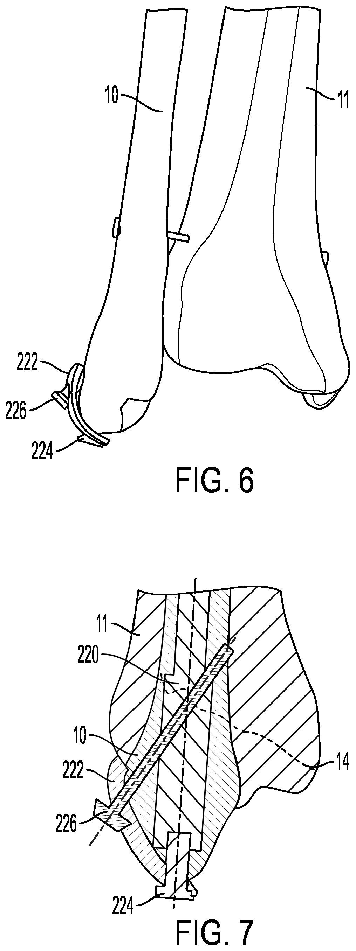

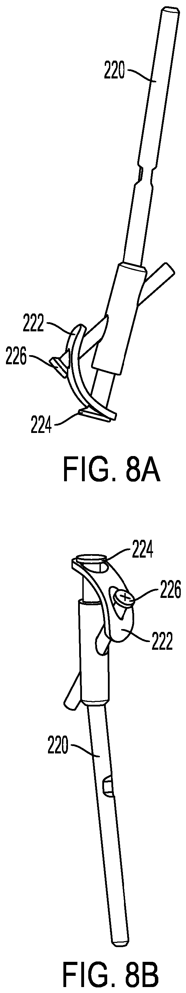

FIGs. 6-8C also depict a similar system to the two foregoing systems that is particularly useful in treating Weber B fractures. Again, because of the similarities between the components of this system with the above-discussed systems, like components are labeled with like reference numerals, but within the 200-series of numbers. For instance, the system of FIGs. 6-8C includes an IM nail 220, a washer 222 and a nail cap 224, the major difference again lying in washer 222, which is an extra extended version. The second hole for reception of a compression screw 226 is located more proximally and permits the compression of the more proximally locate Weber B fracture.

In use of the foregoing systems, IM nail 20, 120, 220 is first inserted into fibula 10 and secured with proximal screws (not shown). This can be accomplished through any known process, for instance, as taught in the surgical techniques pertaining to the aforementioned T2 Alpha and Gamma nails offered by Stryker, the disclosures of which are hereby incorporated by reference herein. Again, IM nail 20, 120, 220 can take many different forms, but in general must be suited for placement in the distal end of fibula 10.

With IM nail 20, 120, 220 in place, washer 22, 122, 222 is placed against the distal-most portion of fibula 10. While in this position, nail cap 24, 124, 224 is placed through the plate and ultimately connected to IM nail 20, 120, 220. This connection may include threading the cap into the distal end of the nail, which causes the reduction of the fractures shown in FIGs. 2, 4 and 7. It also stabilizes distal fragments or avulsion fracture fragments in fibula 10. Of course, it is contemplated to make use of secondary instruments for guiding and accurately placing caps 24, 124, 224.

In the case of washers 122, 222, which are both extended and include a second hole, additional screws 126, 226 can be placed to enhance fixation of the bone fragments. These screws can either lock with or pass through IM nail 120, 220 or be directed into bone without interfering with the nail. It is contemplated that these additional holes in washers 122, 222 can permit angulation of screws placed therethrough, thereby permitting the surgeon to dictate the ultimate placement of the screws. IM nails 120, 220 also include screw holes/slots along their length through which additional screws can be placed, as shown in the drawings.

Washers 22, 122, 222 can be rotated during placement to accommodate fracture-specific requirements or surgeon preferences. It is also contemplated to form the washers of flexible materials, which may permit more conformance to the distal end of fibula 10. Moreover, in addition to the additional holes, the cap holes of washers 22, 122, 222 can be any one of locking, non-locking or hybrid holes allowing for variable angle and different types of screws to be utilized. All of this permits a surgeon to create patient-specific or fracture-specific treatment constructs with minimal effort, while enhancing stability and promoting optimal healing. IM nails 20, 120, 220 can effectively be used for distal fracture management while providing all the benefits of nailing and minimizing plating disadvantages.

While the foregoing discussion focuses on a particular use in a distal fibula, it is to be understood that the IM nail and washer constructs can be useful in connection with other bones and fracture types. Indeed, use on other bones may simply require the washers to be resized and/or shaped to cooperate with those bone surfaces. Of course, other differences in the nails, caps and screws may likewise be necessary.

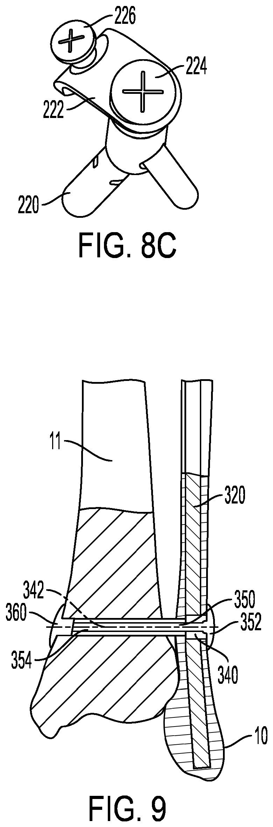

Referring to FIGs. 9-10C, another fibula fixation system is shown and has particular use in treating syndesmotic injuries. This system includes an IM nail 320, a syndesmosis pin 350 and lock washer 360. As shown, IM nail 320 includes a slot 340 through which flexible pin 350 pin passes when implanted. Pin 350 includes an enlarged head 352 and a largely unthreaded shaft 354. Pin 350 can be made of materials like nitinol or the like that are designed to have inherent flexibility. This permits the pins to absorb stress and adapt to loading conditions. In other embodiments, however, the pin need not be inherently flexible. For instance, the pin could be made of a rigid metallic material.

Lock washer 360 is designed to be coupled with pin 350 and effectively creates a second enlarged head that when coupled creates a gradual clamping/compression effect between fibula 10 and tibia 11. Lock washer includes an aperture (not shown) for receiving a portion of shaft 354, via a threaded connection, press fit, riveting, anerobic glue or the like. It is contemplated to employ different types of connections between pin 350 and lock washer 360. Like above discussed washers 22, 122, 222, lock washer 360 is designed to conform to the surface of tibia 11, and thereby provide better stress distribution. This includes constructing the washer of a material suited to conform to the surface upon implantation of the system. The same holds true with respect to head 352 in connection with the surface of fibula 10. It is to be understood that pin 360 can be provided in different lengths and cut, via a pin cutter, to a suitable length.

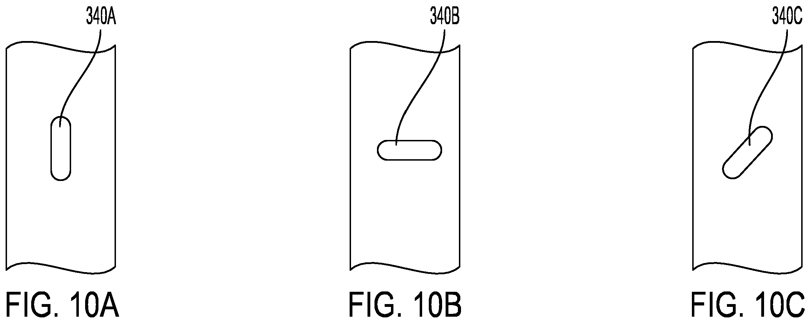

With reference to FIGs. 10A-C, IM nail 320 includes a slot 340, with at least three potential configurations 340A-C of the slot being contemplated for different nails and/or in the same nail. Slot 340A is vertical, slot 340B is horizontal and slot 340C is inclined. This permits a surgeon to select an IM nail 320 with a particular slot or to place pin 350 through a particular hole in a given nail that best corresponds to a desired anatomical alignment. In particular, the elongate nature of slots 340A-C permits some movement of pin 350 therein, which not only facilitates a desired initial positioning, but can permit the pin to move after implantation. This coupled with the flexibility of pin 350 provides a system designed to absorb stress without a failure such as is typically seen with rigidly placed screws. The inclusion of conforming head 352 and lock washer 360 further aids in this function, as such distribute stress more effectively than screw threads.

In use, after proper reduction of the syndesmosis joint and placement of IM nail 320 a channel 342 is created through fibula 10 and tibia 11. This can be accomplished through any known means, including the use of a drill or similar tool. Channel 342 is enlarged to permit some movement of pin 350, which coupled with its flexibility maintains strength while allowing for flexibility. Pin 350 is then inserted from the fibula side through slot 340 and channel 342. Lock washer 360 can then be coupled with pin 350 on the tibia side and gradual compression can be achieved between head 352 and lock washer 360. This creates a secure fixation and one designed to treat the syndesmotic injury. This procedure may require multiple incisions on different sides of the patient's leg. It is contemplated that a bone plate (not shown) could be utilized instead of IM nail 320. In such a case, similar method steps would be followed, however, the plate would include one or more slots 340A-C. It is also contemplated to make use of this system in conjunction with any one of the above-discussed systems when a fibula fracture is present.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A system for treating a syndesmosis injury comprising:
a syndesmosis pin having a head and a shaft; and
an intramedullary nail configured for implantation in a fibula having a slot sized to receive the shaft,
wherein the slot is sized larger than the shaft of the syndesmosis pin to allow for movement of the syndesmosis pin within the slot.

2. The system of claim 1, wherein the syndesmosis pin is flexible.

3. The system of claim 2, wherein the syndesmosis pin is constructed of nitinol.

4. The system of claim 1, wherein a longitudinal axis of the slot is parallel to a longitudinal axis of the intramedullary nail.

5. The system of claim 1, wherein the longitudinal axis of the slot is perpendicular to the longitudinal axis of the intramedullary nail.

6. The system of claim 1, wherein the longitudinal axis slot is neither perpendicular nor parallel to the longitudinal axis of the intramedullary nail.

7. The system of claim 1, wherein the shaft of the syndesmosis pin is unthreaded.

8. The system of claim 7, wherein a medial tip of the syndesmosis pin is threaded.

9. The system of claim 1, further comprising a lock washer configured to be coupled with the syndesmosis pin.

10. The system of claim 9, wherein the lock washer is configured to conform to a surface of a tibia.

11. A system for treating a fracture of a fibula comprising:
an intramedullary nail configured for implantation in a fibula;
a washer configured to cooperate with a distal end of the fibula, the washer including a first hole; and
a nail cap extending through the first hole and connected to the intramedullary nail.

12. The system of claim 11, wherein the washer conforms to the distal end of the fibula.

13. The system of claim 11, wherein the washer includes a second hole configured to receive a bone screw.

14. The system of claim 11, wherein the washer includes a hook for engaging the fibula.

15. The system of claim 11, wherein the nail cap is threaded to a distal end of the intramedullary nail.
